# EUROPÄISCHE PATENTSCHRIFT

(11) **EP 0 305 662 B1**
(45) Veröffentlichungstag und Bekanntmachung des Hinweises auf die Patenterteilung: **24.11.1993**
(21) Anmeldenummer: 88109509.5
(22) Anmeldetag: 15.06.1988
(51) Int. Cl.: C07D 251/38

(54) **Trinatriumsalz des 2,4,6-Trimercapto-s-triazin-nonahydrats und Verfahren zur Herstellung**
Trisodium salt of 2,4,6-trimercapto-s-triazine nonahydrate, and preparation method
Sel trisodium de la 2,4,6-trimercapto-s-triazine nonahydrate et procédé de préparation

(30) Priorität: 31.08.1987 DE 3729029
(43) Veröffentlichungstag der Anmeldung: 08.03.1989
(73) Patentinhaber: Degussa Aktiengesellschaft, 60311 Frankfurt (DE)
(72) Erfinder: Weber, Karl-Ludwig, Dr., B-2080 Kapellen (BE); van Raemdonck, Edwin, B-2750 Beveren (BE); Stützel, Klaus, D-6000 Frankfurt am Main (DE); Vingehoets, Marcel, B-2168 Brecht (BE)

(56) Entgegenhaltungen:
- DE-A- 2 240 733
- DE-A- 3 140 026
- CHEMICAL ABSTRACTS, Band 81, Nr. 1, 8. Juli 1974, Seite 326, Zusammenfassung Nr. 3972b, Columbus, Ohio, US

## Beschreibung

Die Erfindung betrifft das in kristalliner Form vorliegende Trinatriumsalz des 2,4,6-Trimercapto-s-triazin-nonahydrats (TMT-Na₃.9H₂O) und ein Verfahren zur Herstellung.

Das Mononatriumsalz des 2,4,6-Trimercapto-s-triazins wurde bereits von A.W. Hofmann beschrieben (Chem. Ber. 18 (1885) 2196 - 2207). Er stellte diese hygroskopische Verbindung durch Schmelzen von 2,4,6-Trimercapto-s-triazin-tri-s-methylester oder von 2,4,6-Trichlor-s-triazin (Cyanurchlorid) mit Natriumsulfid her.

Ein Trinatriumsalz konnte so nicht gewonnen werden, wie die quantitative Analyse ergab.

Nakamura et al. (C.A. 81, 3972b) beschreibt die Herstellung des Mononatriumsalzes in Form des Trihydrats durch Umsetzung von NaSH und Na₂S mit Cyanurchlorid in wässriger Lösung.

Aus der DE-AS 2 240 733 kann man ein Verfahren zur Fixierung von Schwermetallionen im Erdreich entnehmen, bei dem u. a. auch die wässrige Lösung des Trinatriumsalzes von 2,4,6-Trimercapto-s-triazin verwendet wird.

Ein Weg zur Herstellung dieser Verbindung wird jedoch nicht angegeben.

Die DE-OS 31 400 26 betrifft ein Verfahren, bei dem man z. B. Cu-Ionen aus für die Herstellung von Polyoxyphenylenen benötigten Katalysatoren mit Hilfe einer 15 %igen wässrigen lösung des Trinatriumsalzes entfernt.

Derartige wässrige Lösungen sind im Handel erhältlich.

Entwässert man diese Lösungen, erhält man ein hellgelbes hygroskopisches TMT-Na₃, das nach Schwefelwasserstoff riecht.
(J. Mangels, Diplomarbeit Universität Hamburg 1985).

Nach Hofmann kann man ein Natriumsalz auch aus dem freien 2,4,6-Trimercapto-s-triazin durch Umsetzung mit Natronlauge gewinnen (a.a.O. S. 2200), ohne daß das freie Salz jedoch isoliert wurde.

Gegenstand der Erfindung ist das in kristalliner Form vorliegende Trinatriumsalz des 2,4,6-Trimercapto-s-triazin-nonahydrats.

Ebenfalls Gegenstand der Erfindung ist ein Verfahren zur Herstellung dieser Verbindung, daß dadurch gekennzeichnet ist, daß man Cyanurchlorid im wässrigen Medium bei Temperaturen von 20 bis 70 °C mit NaSH oder Na₂S oder einem NaSH/Na₂S-Gemisch umsetzt, wobei die Gesamtmenge des Schwefels der dreifachen molaren Menge des Cyanurchlorids entspricht und während der Reaktion der pH-Wert-gegebenfalls durch Zusatz von Natronlauge oberhalb-von 7 gehalten wird, im Anschluß an die Reaktion das erhaltene Gemisch auf 0 bis 20 °C abkühlt und das auskristallisierte TMT-Na₃.9H₂O abtrennt.

Bevorzugt führt man die Reaktion bei 40 bis 50 °C und einem pH-Wert von 9 bis 10 durch.

Vor dem Abkühlen des Reaktionsgemisches setzt man diesem vorzugsweise noch soviel Natriumhydroxidlösung zu, bis das gesamte eingesetzte Natriumhydroxid in äquimolarer Menge in der Ausgangskonzentration des NaSH in dem Reaktionsgemisch vorliegt.

Dabei ist die Wassermenge vorteilhaft so zu bemessen, daß eine 15 bis 25 Gew.-%ige, insbesondere 19 bis 21 Gew.-%ige, TMT-Na₃-Lösung entsteht.

Als schwefelhaltige Komponente dienen NaSH oder Na₂S oder NaSH und Na₂S im Gemisch, bevorzugt in einem Molverhältnis Na₂S/NaSH von 0 bis 1:4, insbesondere von 1:2 bis 1:4.

Die Abtrennung des auskristallisierten Produkts erfolgt nach dem Abkühlen in an sich bekannter Weise.

Bevorzugt zentrifugiert man den Feststoff ab und erhält einen Filterkuchen, der neben einem Kristallwasseranteil von ca. 40 Gew.-% noch ca. 5 Gew.-% Restfeuchte enthält.

Die mit dieser Restfeuchte eingeschleppten Verunreinigungen -vor allem Natriumchlorid- bilden einen so geringen Anteil, daß man, bezogen auf den Feststoffgehalt, TMT-Na₃.9H₂O mit einer Reinheit von > 97 % erhält.
Das war nicht zu erwarten gewesen.
Das Produkt ist rein weiß und geruchsfrei.

Die nicht als Kristallwasser vorliegende Feuchtigkeit kann schonend abgetrocknet werden.

Das resultierende kristalline Material zeigt eine gute Rieselfähigkeit und überrascht durch seine Lagerstabilität an Luft bei Umgebungstemperatur. So ist nach zweimonatiger Lagerung keine Änderung der Eigenschaften oder des Gehaltes (HPLC-Analyse) feststellbar.

Dagegen hydrolisieren wässrige TMT-Na₃-Lösungen bei Raumtemperatur merklich, während das reine TMT-Na₃ hygroskopisch ist und zur Verbackung neigt.

Mit Hilfe der erfindungsgemäß hergestellten neuen Verbindung wird die Anwendung des Trinatriumsalzes erleichtert, da man jetzt wässrige Lösungen aus dem in lagerfähiger kristalliner Form vorliegenden Trinatriumsalz vor Ort je nach Bedarf herstellen kann.

In einer bevorzugten Ausführungsform des Verfahrens arbeitet man das bei der Abtrennung des kristallisierten Produkts anfallende Filtrat auf.

Dieses wird bis auf pH2-4 angesäuert, bevorzugt mit Salzsäure.

Das ausfallende 2,4,6-Trismercapto-s-triazin (TMT-H₃) wird abgetrennt und ohne Trocknung in eine Natriumhydroxidlösung eingetragen, deren Konzentration so gewählt wird, daß eine 30 - 40 Gew.-%ige TMT-Na₃-Lösung entsteht.

Das sich dabei bildende Trinatriumsalz kristallisiert bei der Abkühlung auf 0 bis 20 °C aus und wird abfiltriert, während man das anfallende Filtrat mit einem Gehalt von ca. 15 - 20 Gew.-% TMT-Na₃ als wässriges Medium für die Reaktion von Cyol mit NaSH und/oder Na₂S einsetzen kann.

Einfacher ist es noch, das TMT-H₃ direkt auch in diese Reaktionsstufe zurückzuführen, was dann natürlich bei der Zugabe des Natriumhydroxids entsprechend berücksichtigt werden muß.

Damit erreicht man eine Gesamtausbeute, bezogen auf Cyanurchlorid von mindestens 90 %.

### Beispiele

### Beispiel 1:

172 g 40 Gew.-%ige wässrige NaSH (1,23 Mol) werden mit 112 ml Wasser verdünnt. Dazu werden bei 50 °C 75 g Cyanurchlorid (0,41 Mol) und 60 g 50 Gew.-%ige wässrige NaOH (0,75 Mol) so dosiert, daß der pH-Wert zwischen 9,5 und 10,5 und die Temperatur bei 50 °C gehalten wird. Nach beendeter Zugabe wird noch 1 h bei 50 °C gerührt und anschließend durch Zufügen von 39 g 50 Gew.-%iger wässriger NaOH (0,48Mol) der pH-Wert auf 12,5 gestellt. Nach dem Kühlen auf 10 °C wird zentrifugiert.
Der Filterkuchen wiegt 146 g und enthält 44,5 Gew.-% H₂O, 54,1 Gew.-% TMT-Na₃ und 1,3 Gew.-% NaCl. Der TMT-Na₃-Gehalt entspricht einer theoretischen Ausbeute von 79,3 %, bezogen auf eingesetztes Cyol.

Das Filtrat wiegt 31 2g und enthält 5,2 Gew.-% TMT-Na₃, was einer theoretischen Ausbeute von 16,2 %, bezogen auf eingesetztes Cyol bedeutet. Im Filtrat befinden sich ferner ca. 22,7 Gew.-% NaCl und in geringem Maße Hydrolyseprodukte des Cyanurchlorids.

Das Filtrat wird mit 32 g konzentrierter Salzsäure angesäuert, und das dabei quantitativ ausgefällte TMT-H₃ filtriert und mit Wasser gewaschen.
Der feuchte Filterkuchen enthält 11,7 g TMT-H₃- 100 %.

### Beispiel 2:

15 g Na₂S (0,19 Mol) und 48,0 g NaSH (0,8 Mol) werden in 133 ml Wasser vorgelegt und bei 40 °C 65,0 g Cyanurchlorid (0,35 Mol) so zugefügt, daß die Temperatur gehalten wird. Der pH-Wert sinkt zunächst auf 9 und wird dort durch gleichzeitige Zugabe einer 30 Gew.-%igen, wässrigen NaOH gehalten bis sämtliches Cyanurchlorid zugefügt ist. Nach einer halben Stunde Nachreaktionszeit wird der pH-Wert durch Zufügen des Restes der insgesamt 115 g Natronlauge (0,86 Mol) auf 12,5-13 erhöht. Die Aufarbeitung erfolgt wie gewohnt. Es werden 124 g TMT-Na₃-Filterkuchen mit einem NaCl-Gehalt von 1,4 Gew.-%, einem Wassergehalt von 44,9 Gew.-% und einem TMT-Na₃-Gehalt von 53,2 Gew.-%, was einer Ausbeute von 77,6 % bezogen auf Cyanurchlorid bedeutet, erhalten. Die 252 g Filtrat mit 5,1 Gew.-% TMT-Na₃ - entspricht 15,1 % Ausbeute bezogen auf Cyanurchlorid - und mit 23,5 Gew.-% NaCl werden mit 20 g konzentrierter Salzsäure behandelt. Dabei wird wie gewohnt der TMT-H₃-Filterkuchen isoliert. Er enthält 9,3 g TMT-H₃ - 100 %.

### Beispiel 3:

69 g TMT-H₃-Filterkuchen mit einem TMT-H₃-Gehalt von 45 Gew.-% (0,17 Mol) und einem Wassergehalt von 55 Gew.-% werden zu 70 g einer 30 Gew.-%igen, wässrigen NaOH (0,52 Mol) gegeben, wobei die Temperatur durch Kühlung zwischen 40 und 50 °C gehalten wird. Die klare Lösung wird auf 10 °C gekühlt, wobei TMT-Na₃ kristallisiert, das wie gewohnt abgetrennt wird. Der zentrifugierfeuchte Filterkuchen wiegt 46,0 g und enthält 56,5 Gew.-% TMT-Na₃ (0,10 Mol) und 43,5 Gew.-% Wasser.

Das Filtrat, das 93 g wiegt und 18 Gew.-% TMT-Na₃ (0,07 Mol) enthält, wird mit 56 g NaSH (1 Mol) und 136 g Wasser versetzt. Analog zu Beispiel 1 erfolgt die Reaktion mit 62,0 g Cyol (0,33 Mol) und 80 g 50 Gew.-%ige wässrige NaOH (1 Mol) und die Aufarbeitung. Der 139 g schwere Filterkuchen enthält 1,1 Gew.-% NaCl, 44,1 Gew.-% Wasser und 54,5 Gew.-% TMT-Na₃.
Letzterer entspricht einer Ausbeute von 78,0 % an TMT-Na₃,bezogen auf eingesetzes TMT-Na₃ und Cyanurchlorid. Das Filtrat, das 288 g wiegt und 19,5 Gew.-% NaCl und 6,1 Gew.-% TMT-Na₃, was einer Ausbeute von 18,1 % an TMT-Na₃ bezogen auf eingesetztes TMT-Na₃ und Cyanurchlorid entspricht, enthält, wird mit 37 g konzentrierter Salzsäure versetzt und TMT-H₃ isoliert.

### Beispiel 4:

Bei 40 bis 50 °C wird in einer Lösung von 36 g NaSH (0,64 Mol) und 20,0 g Na₂S (0,26 Mol) in 214 g Wasser portionsweise 33 g TMT-H₃-Filterkuchen mit einem H₂O-Gehalt von 65 % und einem TMT-H₃-Gehalt von 45 Gew.-% (0,08 Mol) zugeführt. Anschließend wird wie gewohnt die Reaktion bei 50 °C und einem pH-Wert von 9 -10 mit 55,0 g Cyol (0,30 Mol) und 71 g 50 Gew.-%ige, wässrige NaOH (0,90 Mol) durchgeführt und aufgearbeitet. Die Ausbeute an TMT-Na₃, bezogen auf eingesetzes Cyanurchlorid und TMT-H₃, beträgt im Filterkuchen 69,8 % und im Filtrat 23,2 %. Das Filtrat- 310 g mit 6,9 Gew.-% TMT-Na₃,15 Gew.-% NaCl - wird mit 32 g konzentrierter Salzsäure angesäuert und ausgefallenes TMT-H₃ abgetrennt. Der TMT-Na₃-Filterkuchen wiegt 119 g und enthält 44,7 Gew.-% H₂O, 0,8 Gew.-% NaCl und 54,2 Gew.-% TMT-Na₃

## Patentansprüche

1. In kristalliner Form vorliegendes Trinatriumsalz des 2,4,6-Trimercapto-s-triazinnonahydrats (TMT-Na₃.9H₂O)

2. Verfahren zur Herstellung der Verbindung gemäß Anspruch 1, dadurch gekennzeichnet, daß man Cyanurchlorid in wässrigem Medium bei Temperaturen von 20 bis 70 °C mit NaSH oder Na₂S oder einem NaSH/Na₂S-Gemisch umsetzt, wobei die Gesamtmenge des Schwefels der dreifachen molaren Menge des Cyanurchlorids entspricht und während der Reaktion der pH-Wert-gegebenenfalls durch Zusatz von Natronlauge-oberhalb von 7 gehalten wird, im Anschluß an die Reaktion das erhaltene Gemisch auf 0 °C bis 20 °C abkühlt und das auskristallisierte TMT-Na₃.9H₂O abtrennt.

3. Verfahren gemäß Anspruch 2, dadurch gekennzeichnet, daß man dem Reaktionsgemisch vor dem Abkühlen soviel Natriumhydroxidlösung zusetzt, daß das gesamt eingesetzte Natriumhydroxid in äquimolarer Menge zu der NaSH-Ausgangskonzentration vorliegt.

4. Verfahren gemäß den Ansprüchen 2 und 3, dadurch gekennzeichnet, daß man die Wassermenge im Reaktionsgemisch so bemißt, daß man eine 15 bis 25 Gew.-%ige TMT-Na₃-Lösung erhält.

5. Verfahren gemäß den Ansprüchen 2 bis 4, dadurch gekennzeichnet, daß man aus dem bei der Abtrennung des TMT-Na₃.9H₂O anfallenden Filtrat durch Ansäuern das 2,4,6-Trimercapto-s-triazin (TMT-H₃) ausfällt und dieses entweder direkt in das TMT-Na₃.9H₂O umwandelt oder in das Ausgangsreaktionsgemisch zurückführt.

## Claims

1. Trisodium salt of 2,4,6-trimercapto-3-triazine nonahydrate (TMT-Na₃.9H₂O) in crystalline form.

2. A process for the preparation of the compound according to Claim 1, characterised in that cyanuric chloride is reacted with NaSH or Na₂S or an NaSH/Na₂S mixture in an aqueous medium at temperatures of from 20 to 70°C, the total quantity of the sulphur being equal to three times the molar quantity of the cyanuric chloride and the pH being maintained above 7 during the reaction, if necessary by the addition of sodium hydroxide solution, and the mixture obtained after the reaction is cooled to a temperature from 0°C to 20°C and the crystallised TMT-Na₃.0H₂O is separated.

3. A process according to Claim 2, characterised in that before the reaction mixture is cooled, sodium hydroxide solution is added in such a quantity that the total quantity of sodium hydroxide used is equimolar to the NaSH starting concentration.

4. A process according to Claims 2 and 3, characterised in that the quantity of water in the reaction mixture is calculated to provide a 15 to 25% by weight TMT-Na₃ solution.

5. A process according to Claims 2 to 4, characterised in that the 2,4,6-trimercapto-S-triazine (TMT-H₃) is precipitated by acidification from the filtrate obtained on removal of the TMT-Na₃.9H₂O and is either directly converted into TMT-Na₃.9H₂O or returned to the starting reaction mixture.

## Revendications

1. Sel trisodique de la 2,4,6-trimercapto-s-triazine nonahydrate (TMT-Na₃.9H₂O) sous forme cristalline.

2. Procédé de préparation du composé de la revendication 1, caractérisé en ce qu'on fait réagir le chlorure de cyanuryle en milieu aqueux à des températures comprises entre 20 et 70°C avec NaSH ou Na₂S ou un mélange NaSH/Na₂S, la quantité totale de soufre correspond à trois fois la quantité molaire du chlorure de cyanuryle et pendant la réaction on maintient le pH le cas échéant par addition de solution de soude au-dessus de 7, après la fin de la réaction on refroidit le mélange obtenu entre 0 et 20°C et on sépare la TMT-Na₃.9H₂O qui a cristallisé.

3. Procédé selon la revendication 2, caractérisé en ce qu'on ajoute au mélange réactionnel avant le refroidissement suffisamment de solution d'hydroxyde de sodium, pour que la quantité totale d'hydroxyde de sodium utilisée soit en quantité équimolaire par rapport à la concentration de départ du NaSH.

4. Procédé selon les revendications 2 et 3, caractérisé en ce qu'on ajuste la quantité d'eau dans le mélange réactionnel de façon à obtenir une solution de 15 à 25 % en poids de TMT-Na₃.

5. Procédé selon les revendications 2 à 4, caractérisé en ce qu'on fait précipiter par acidification la 2,4,6-trimercapto-s-triazine (TMT-H₃) à partir du filtrat obtenu lors de la séparation de la TMT-Na₃.9H₂O et que soit on la transforme directement en TMT-Na₃.9H₂O, soit on la recycle dans le mélange réactionnel de départ.
